# EUROPEAN PATENT APPLICATION

(11) **EP 3 978 925 A1**
(43) Date of publication of application: **06.04.2022**
(21) Application number: 20812503.9
(22) Date of filing: 10.01.2020
(51) Int. Cl.: G01N 33/543, G01N 33/566, B01J 20/281, B01D 15/38

(54) **AFFINITY SEPARATION SYSTEM AND METHOD USING SWITCH-LIKE ADHESION REACTION**

(30) Priority: 24.05.2019 KR 20190061120; 16.08.2019 KR 20190100142
(71) Applicant: Sol Bio Corporation, Seoul 04780 (KR)
(72) Inventor: PAEK, Se Hwan, Seoul 04971 (KR)
(74) Representative: Hoefer & Partner Patentanwälte mbB
(86) International application number: PCT/KR2020/000492
(87) International publication number: WO 2020/241999

(57) **Abstract**

The present invention relates to an affinity isolation system and method using a switch-like binding reaction. An affinity isolation system according to an embodiment of the present invention comprises: a ligand (10); a binder (20) to which the ligand (10) is reversibly bound and which structurally changes with the binding reaction thereof with the ligand (10); a recognition material (30) specifically binding to the structurally changed binder (20); an immobilizing member (40) having a surface onto which any one of the binding pair, the binder (20) and the recognition material (30), is immobilized; and a capturing material (50) conjugating with the other of the binding pair, the binder (20) and the recognition material (30), to form a conjugate (C) and specifically binding to the target (1) in a target subpopulation from a sample solution.

## Description

### Technical Field

The present invention relates to an affinity isolation system and method using switch-like binding, and more specifically to a technology for isolating target components in a sample into a subpopulation.

### Background Art

Recently, the concept of "liquid biopsy" has been introduced to minimize pain during screening for specific diseases such as cancer and degenerative diseases and to achieve early diagnosis of the diseases. Liquid biopsy refers to a non-invasive method for extracting body fluids, including blood, saliva, and urine, in a relatively simple manner and analyzing specific disease-associated cells or components (e.g., peptides, proteins, nucleic acids, organelles, and specific substances) released therefrom in the body fluids. Liquid biopsy helps to ascertain the occurrence of specific diseases at an early stage. For this reason, liquid biopsy has attracted attention as an alternative to existing test methods such as diagnostic imaging, tissue examination, and blood testing.

Particularly, exosomes are called the avatars of cells because they are released in the form of microvesicles (MVEs) from human cells and reflect the characteristics of the original cells. Exosomes can be extracted from various body fluids such as blood, saliva, and urine. Since exosomes are vesicles composed of lipid bilayers, they are not only stable *in vivo* but also free from the attack of protease, RNase, and DNase. Under these circumstances, there has been increasing research on the use of exosomes as new biomarkers for diagnosing specific diseases such as cancer and degenerative diseases. In practice, products using exosomes have been developed or are currently being developed for early diagnosis of cancer.

However, since exosomes derived from all cells exist in a mixed state in body fluids, there is a limitation in accurately diagnosing specific diseases such as cancer from a bulk population of exosome samples separated from body fluids. Moreover, since the concentration of exosomes derived from specific disease-associated cells in body fluids is relatively very low in the early stage of disease onset, isolation and enrichment of target exosomes from body fluids are prerequisites for early diagnosis. Many techniques for exosome isolation are known. Of these, ultracentrifugation based on a density difference is adopted as the gold standard for exosome isolation due to its high reproducibility and relatively stable process. However, ultracentrifugation has disadvantages in that the use of an expensive system is required and it takes a long time for isolation. In attempts to overcome these disadvantages, methods for isolating exosomes by precipitation and methods for isolating exosomes by size exclusion chromatography or on the basis of size using microfluidics have been developed. For example, a method for isolating exosomes on the basis of size using microfluidics is disclosed in Korean Patent No. 10-1807256.

However, the existing exosome isolation techniques are limited in selectively detecting trace amounts of exosomes derived from specific cells such as cancer cells because all exosomes present in body fluids are provided in the form of single populations.

Thus, there is an urgent need for a solution to the problems of conventional techniques for isolating cells or cell-derived components.

### Detailed Description of the Invention

### Problems to be Solved by the Invention

The present invention has been made in an effort to solve the problems of the prior art and intends to provide a technology for affinity isolation in which a subpopulation of target components is separated in high yield while keeping the target components intact based on switch-like reversible binding using recognition materials capable of specifically recognizing a conformational change of binders such as binding proteins, which is caused when the binders bind to ligands, to allow the binders to rapidly bind to or unbind from the recognition materials in the presence or absence of the ligands in a solution.

### Means for Solving the Problems

An affinity isolation system according to an embodiment of the present invention includes ligands, binders to which the ligands reversibly bind and whose structural conformation is changed upon binding to the ligands, recognition materials specifically binding to the binders whose structural conformation has been changed, immobilization members whose surface is immobilized with either the binders or the recognition materials, and capture materials conjugated with the binders or recognition materials, not used in the immobilization, to form conjugates and specifically binding to targets of a target subpopulation in a sample solution.

The ligands may be selected from the group consisting of sugar molecules, ions, substrates, antigens, and combinations thereof.

The binders may be selected from the group consisting of sugar-binding proteins, ion-binding proteins, enzymes, antibodies, aptamers, cell receptors, nanostructures, and combinations thereof.

The recognition materials may be selected from the group consisting of antibodies, protein receptors, cell receptors, aptamers, enzymes, nanoparticles, nanostructures, heavy metal chelators, and combinations thereof.

The immobilization members may be selected from the group consisting of hydrogels, magnetic beads, latex beads, glass beads, nanometal structures, porous membranes, non-porous membranes, and combinations thereof.

The affinity isolation system may further include a reactor accommodating the immobilization members therein.

The binding complexes of the binders with the recognition materials may be dissociated from each other when the ligands are detached from the binders.

The immobilization members bound to the targets may be concentrated using magnetic force, gravitational force, and/or centrifugal force.

The sample solution may include a body fluid and the targets may be substances isolated from heterogeneous bulk populations in the body fluid.

The heterogeneous bulk populations may include at least two substances selected from the group consisting of cells, exosomes, DNAs, RNAs, proteins, lipids, sugars, and metabolites used as biomarkers to diagnose target diseases.

An affinity isolation method according to an embodiment of the present invention includes (a) serial reactions of a solution containing ligands, binders whose structural conformation is changed upon binding to the ligands, recognition materials specifically binding to the binding partners, *i.e.* the binders whose structural conformation has been changed, and capture materials conjugated with either the binders or the recognition materials to form conjugates such that the counterpart not used in the conjugation, *i.e.* the binders or recognition materials, bind to the conjugates, (b) addition of a sample solution containing targets of a target subpopulation capable of specifically binding to the capture materials such that the capture materials of the conjugates bind to the targets, and (c) addition of a recovery solution without the ligands such that the ligands are detached from the binders, triggering to dissociate the conjugates, initially formed the binding complexes with the targets, from the counterpart not used in the conjugation, *i.e.* the recognition materials or binders.

Step (a) may include immobilization of either of the binders or recognition materials, not used in the conjugation, on the surface of the immobilization members and addition of the ligand solution containing the conjugates dissolved therein to the immobilization members such that the conjugates bind to the counterpart immobilized onto the immobilization members.

The affinity isolation method may further include, between steps (b) and (c), capture of the immobilization members bound to the targets within predetermined spaces of a solution of the ligand mixed with the sample using magnetic force, gravitational force, and/or centrifugal force, and then removal of a portion of the mixed solution, where the immobilization members are absent, to enrich the targets.

The sample solution may include a body fluid and the targets may be substances isolated from heterogeneous bulk populations in the body fluid.

The heterogeneous bulk populations may include at least two substances selected from the group consisting of cells, exosomes, DNAs, RNAs, proteins, lipids, sugars, and metabolites used as biomarkers for the diagnosis of target diseases.

The features and advantages of the present invention will become apparent from the following description with reference to the accompanying drawings.

Prior to the detailed description of the invention, it should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

### Effects of the Invention

According to the present invention, specific disease-associated target biological components can be isolated into a subpopulation under mild conditions. The target subpopulation can be used as a liquid biopsy sample to diagnose specific diseases such as cancer and degenerative diseases with high sensitivity.

### Brief Description of the Drawings

Fig. 1 is a diagram illustrating the construction of an affinity isolation system based on switch-like binding according to an embodiment of the present invention.
Fig. 2 is a diagram illustrating the construction of an affinity isolation system based on switch-like binding according to a further embodiment of the present invention.
Fig. 3 is a process flow diagram illustrating an affinity isolation method based on switch-like binding according to an embodiment of the present invention.
Fig. 4 is a process flow diagram illustrating an affinity isolation method based on switch-like binding according to a further embodiment of the present invention.
Fig. 5 is a flow diagram illustrating a process for separating an exosome subpopulation associated with melanoma cancer cells using an affinity isolation method based on switch-like binding according to an embodiment of the present invention.
Fig. 6 shows the results of immunoaffinity chromatography during isolation of CD63+ exosomes from a sample using an affinity isolation system and method based on switch-like binding according to the present invention.
Fig. 7 shows the reproducibility of exosome isolation when the immunoaffinity chromatography of Fig. 6 was repeated.
Fig. 8 shows the results of immunomagnetic isolation during isolation and enrichment of CD63+ exosomes from a sample using an affinity isolation system and method based on switch-like binding according to the present invention.
Fig. 9 shows the reproducibility of exosome isolation when the immunomagnetic isolation of Fig. 8 was repeated.
Fig. 10 shows the results of physical characterization for CD63+ exosomes isolated using an affinity isolation system and method based on switch-like binding according to the present invention.
Fig. 11 shows the results of immunochemical characterization for exosome surface markers of CD63+ exosome samples isolated using an affinity isolation system and method based on switch-like binding according to the present invention.
Fig. 12 shows the corrected concentrations of Cavl based on the concentration of CD9 to compensate for variations in concentration between exosome standard samples.
Fig. 13 shows the potential effects of the use of CD63+ exosomes, isolated using an affinity isolation system and method based on switch-like binding according to the present invention, as analytical samples on melanoma cancer diagnosis.
Fig. 14 compares the abilities of inventive affinity isolation systems based on switch-like binding to distinguish melanoma cancer samples from normal samples based on a Cav1/CD9 concentration ratio determined by immunoassay of the separated samples with those of currently commercially available exosome isolation systems.

### Mode for Carrying out the Invention

The objects, specific advantages, and novel features of the present invention will become more apparent from the following detailed description and preferred embodiments, examples of which are illustrated in the accompanying drawings. The same reference numerals and signs denote the same or like components even when they are shown in different accompanying drawings from one another. Although the terms "first", "second", etc. are used herein to describe various components, these components should not be limited by these terms. These terms are only used to distinguish one component from another component. Lest it should obscure the subject matter of the invention, a detailed description of well-known technologies is avoided.

Preferred embodiments of the present invention will now be described in detail with reference to the accompanying drawings.

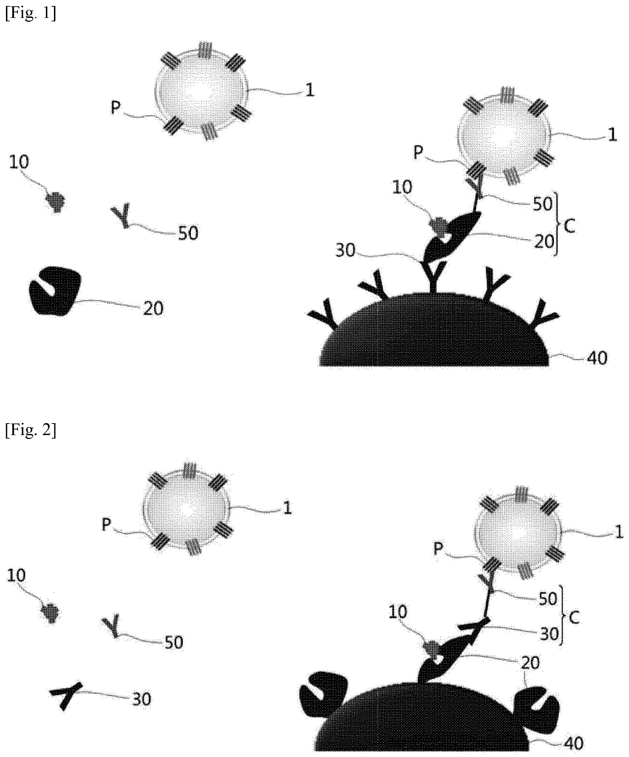

Fig. 1 is a diagram illustrating the construction of an affinity isolation system based on switch-like binding according to an embodiment of the present invention and Fig. 2 is a diagram illustrating the construction of an affinity isolation system based on switch-like binding according to a further embodiment of the present invention.

Each of the affinity isolation systems illustrated in Figs. 1 and 2 includes ligands 10, binders 20 to which the ligands reversibly bind and whose structural conformation is changed upon binding to the ligands 10, recognition materials 30 specifically binding to the binders 20 whose structural conformation has been changed, immobilization members 40 whose surface is immobilized with either the binders 20 or the recognition materials 30, and capture materials 50 conjugated with the binders 20 or recognition materials 30, not used in the immobilization, to form conjugates C and specifically binding to targets 1 of a target subpopulation in a sample solution.

The present invention is directed to a technology for isolating target components in a sample into a subpopulation. Liquid biopsy is a diagnostic method that can be used for early determination of the occurrence of a specific disease by drawing a body fluid such as blood, saliva or urine and subsequently analyzing components (e.g., peptides, proteins, nucleic acids, organelles, and other specific substances) released from specific disease-associated cells. In particular, exosomes derived from the body fluid have been intensively studied as a novel biomarker for the diagnosis of specific diseases such as cancers and degenerative diseases. However, since exosomes derived from all cells exist in a mixed state in body fluids, there is a limitation in accurately diagnosing specific diseases such as cancer using exosome samples in the form of a bulk population merely separated from body fluids. Thus, there is a need to develop a technique for isolating and enriching target exosomes. Conventional techniques for exosome isolation are based on ultracentrifugation, precipitation separation, size exclusion chromatography or microfluidics. However, as these techniques provide all exosomes of a body fluid as sample in the form of a mixed bulk population, there is a limitation in selectively detecting trace amounts of target exosomes derived from specific cells such as cancer cells.

Thus, the present invention employs immunoaffinity isolation for specific markers to separate a specific exosome subpopulation, based on the finding that protein markers highly correlated with cancer, for example, Caveolin 1 (Cavl) for melanoma cancer, HER-2/neu for gastric cancer, and L1CAM for ovarian cancer are present on the surface of exosomes. In contrast, existing general processes for isolating and detecting trace amounts of exosomes in body fluids have difficulty in overcoming the limit of detection and maintaining reproducibility. In practice, since harsh conditions such as acidic pH and/or a surfactant are used to recover specific exosomes captured on the surfaces of solid matrices, there are still problems such as damage to exosomes and low yield of recovery. The present invention has been made in view of the above problems and provides an affinity isolation technology for recovering a target subpopulation from a sample in high yield without using harsh conditions while keeping the targets intact.

The sample is a body fluid for liquid biopsy and the targets 1 may be exosomes but are not necessarily limited thereto. The use of the body fluid is not necessarily limited to liquid biopsy and the sample may be any type that contains the targets 1. Alternatively, the targets 1 may be cells or cell-derived components and are not particularly limited as long as they can be isolated by the present invention.

Specifically, the affinity isolation system of the present invention includes ligands 10, binders 20, recognition materials 30, immobilization members 40, and capture materials 50.

The ligands 10 are materials capable of reversibly binding to the binders 20 and may be selected from the group consisting of sugar molecules, ions, substrates, antigens, and combinations thereof.

The binders 20 are materials whose structural conformation is changed upon binding to the ligands 10. The binders 20 may be selected from the group consisting of sugar-binding proteins, ion-binding proteins, enzymes, antibodies, aptamers, cell receptors, nanostructures, and combinations thereof. Accordingly, the binders 20 and the ligands 10 can form binder-ligand pairs such as sugar-binding protein-sugar molecule pairs, ion-binding protein-ion pairs, enzyme-substrate pairs, antigen-antibody pairs, aptamer-ligand pairs, cell receptor-ligand pairs or nanostructure-ligand pairs. The most representative examples of the binders 20 and the ligands 10 include calcium-binding proteins (CBPs) and calcium ions, respectively. However, the binders 10 and the ligands 10 are not necessarily limited to the above-mentioned materials and may be any materials that can reversibly bind each other to cause their conformational changes.

The recognition materials 30 are materials that specifically bind to the binders 20 whose structural conformation has been changed upon binding to the ligands 20. The recognition materials 30 may be selected from the group consisting of antibodies, protein receptors, cell receptors, aptamers, enzymes, nanoparticles, nanostructures, heavy metal chelators, and combinations thereof. However, these materials are merely illustrative and the scope of the present invention is not necessarily limited thereto. For example, calcium ions as the ligands 10 primarily bind to calcium binding proteins as the binders 20 to cause a conformational change of the calcium binding proteins, and the calcium binding proteins whose structural conformation has been changed react and bind to antibodies as the recognition materials 30 ("antigen-antibody reaction").

Either of the binders 20 and the recognition materials 30 are immobilized onto the immobilization members 40, and the binders 20 or recognition materials 30, not used in the immobilization, are conjugated with the capture materials 50 to form conjugates C. That is, the recognition materials 30 are immobilized onto the immobilization members 40 and the binders 20 are conjugated with the capture materials 50 to form conjugates C, as illustrated in Fig. 1 (see the first embodiment and Examples 2 and 3). Alternatively, the binders 20 are immobilized onto the immobilization members 40 and the recognition materials 30 are conjugated with the capture materials 50 to form conjugates C, as illustrated in Fig. 2 (see the second embodiment).

In each of the first and second embodiments, the binders 20 or the recognition materials 30 are immobilized on the surface of the immobilization members 40. The immobilization members 40 may be selected from the group consisting of, but not necessarily limited to, hydrogels (see Example 2), magnetic beads (see Example 3), latex beads, glass beads, nanometal structures, porous membranes, non-porous membranes, and combinations thereof. However, any materials that can bind with the binders 20 or the recognition materials 30 may be used without particular limitation for the immobilization members 40. The capture materials 50 are substances such as antibodies that are conjugated with the binders 20 or the recognition materials 30, and specifically bind to the targets 1. The capture materials 50 such as antibodies can specifically bind to target markers P (*e.g.,* surface proteins) of the targets 1, for example, through an antigen-antibody reaction.

In the first embodiment, the recognition materials 30 are immobilized onto the immobilization members 40, the binders 20 whose structural conformation has been changed by binding to the ligands 10 react with the recognition materials 30, and the capture materials 50 conjugated with the binders 20 specifically bind to and capture the targets 1. Here, the binders 20 are conjugated with the capture materials 50 to form the conjugates C and then bind to the ligands 10. Alternatively, the binders 20 bind to the ligands 10 and are then conjugated with the capture materials 50.

In the second embodiment, the binders 20 are immobilized onto the immobilization members 40, the recognition materials 30 bind to the binders 20 whose structural conformation has been changed by binding to the ligands 10, and the capture materials 50 conjugated with the recognition materials 30 specifically bind to and capture the targets 1. Here, the conjugates C of the recognition materials 30 with the capture materials 50 react with the binders 20. Alternatively, the recognition materials 30 react with the ligands 10 and are then conjugated with the capture materials 50.

In the first and second embodiments, each binding reaction may be performed in a reactor (not illustrated) accommodating the immobilization members 40 therein. The immobilization members 40 capture the targets 1 via mediation of the recognition materials 30, the binders 20, and the capture materials 50 within the reactor. Here, the targets 1 can be enriched using magnetic force, gravitational force, and/or centrifugal force. For example, when magnetic beads are used as the immobilization members 40, a magnet is used to magnetically capture the immobilization members 40 already reacted with the targets 1. Then, the supernatant is discarded and the magnetic beads are removed to enrich the targets.

The captured targets 1 can be recovered from the magnetic beads by detaching the ligands 10 from the binders 20 to restore the changed conformation of the binders 20 to their original state so that the binding complexes of the binders 20 with the recognition materials 30 are dissociated from each other. Here, the ligands 10 can be detached by the addition of a recovery solution without the ligands 10.

Due to its construction, the affinity isolation system of the present invention can be used to isolate and recover cells or cell-derived components associated with a specific disease such as cancer or degenerative disease from a body fluid. In this case, the sample solution contains a body fluid in which heterogeneous bulk populations are present. The affinity isolation system can be used to isolate and recover targets from heterogeneous bulk populations. The heterogeneous bulk populations may include at least two substances selected from the group consisting of cells, exosomes, DNAs, RNAs, proteins, lipids, sugars, and metabolites used as biomarkers to diagnose target diseases.

Overall, the present invention is based on the use of the recognition materials 30 (*e.g.*, antibodies) capable of specifically recognizing a conformational change of the binders 20 (*e.g.,* calcium binding proteins) caused by binding to the ligands 10 (*e.g.,* calcium ions) specifically reacting with the binders 20. The reaction between the binders 20 and the recognition materials 30 may be an antigen-antibody reaction in which the binders 20 quickly associate with the recognition materials 30 or dissociate from the binding complexes between the two components depending on the presence or absence of the ligands 10 such as calcium ions. This association or dissociation reaction is described as "switch-like reversible binding" because its principle is similar to that of light turning on/off by switching. According to the present invention, a target subpopulation including target markers can be separated and concentrated with high efficiency under mild conditions based on a new binding reaction such as a reversible antigen-antibody reaction. That is, the present invention enables the recovery of targets (*e.g.*, exosomes) of a target subpopulation in high yield while maintaining the structural integrity of the targets in comparison with approaches that use harsh conditions such as acidic pH to recover targets (*e.g*., exosomes) of target subpopulations bound to the surface of solid beads via antigen-antibody reactions.

A description will be given below regarding an affinity isolation method using an affinity isolation system according to the present invention. As the affinity isolation system has been described above, a repeated explanation thereof is omitted or simplified.

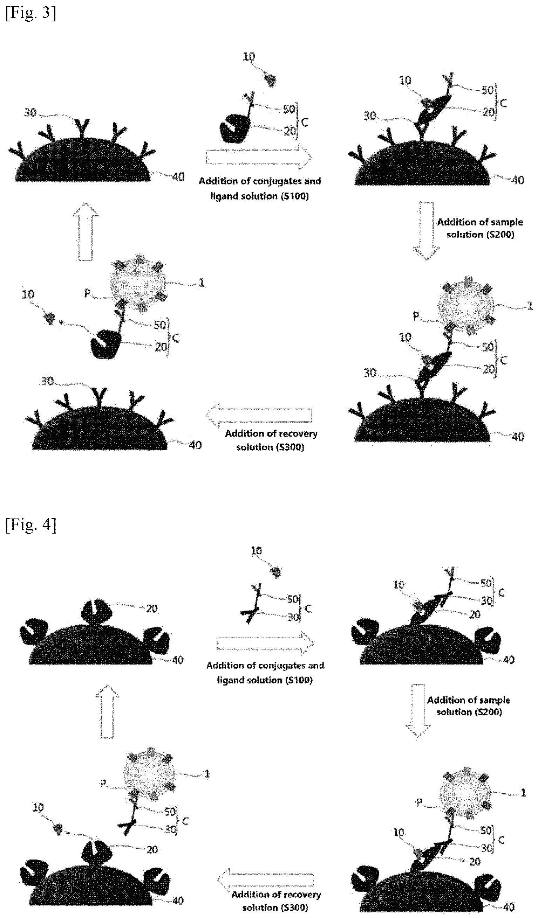

Fig. 3 is a process flow diagram illustrating an affinity isolation method based on switch-like binding according to an embodiment of the present invention and Fig. 4 is a process flow diagram illustrating an affinity isolation method based on switch-like binding according to a further embodiment of the present invention.

Each of the affinity isolation methods illustrated in Figs. 3 and 4 includes (a) serial reactions of a solution containing ligands 10, binders 20 whose structural conformation is changed upon binding to the ligands 10, recognition materials 30 specifically binding to the binders whose structural conformation has been changed, and capture materials 50 conjugated with either the binders 20 or the recognition materials 30 to form conjugates C such that the counterpart not used in the conjugation, *i.e.* the binders 20 or recognition materials 30, bind to the conjugates C (S100), (b) addition of a sample solution containing targets 1 of a target subpopulation capable of specifically binding to the capture materials 50 such that the capture materials 50 of the conjugates C bind to the targets 1 (S200), and (c) addition of a recovery solution without the ligands 10 such that the ligands 10 are dissociated from the binders 20, triggering to separate the conjugates C, formed the binding complexes with the targets 1, from the counterpart not used in the conjugation, *i.e.* the recognition materials 30 or binders 20 (S300).

Step (a) may include immobilization of the binders or recognition materials, not used in the conjugation, on the surface of the immobilization members and addition of the ligand solution containing the conjugates including the binding counterpart dissolved therein to the immobilization members such that the conjugates bind to the binders or recognition materials immobilized onto the immobilization members.

The affinity isolation method may further include, between steps (b) and (c), capture of the immobilization members bound to the targets within predetermined spaces of a solution of the ligand mixed with the sample using magnetic force, gravitational force, and/or centrifugal force, and then removal of a portion of the mixed solution, where the immobilization members are absent, to enrich the targets.

For isolation and recovery of targets 1 of a target subpopulation in a sample solution, first, ligands 10, binders 20, recognition materials 30, and capture materials 50 are allowed to react in a solution with one another (S100). As a result of the serial reactions, as either the binders 20 or the recognition materials 30 are conjugated with the capture materials 50 to form conjugates C, the binders 20 or recognition materials 30, not used in the conjugation, bind to the conjugates C. Here, ligands 10 present in the ligand solution bind to the binders 20 to cause a conformational change of the binders 20 and the recognition materials 30 specifically recognize and bind to the binders 20 whose structural conformation has been changed.

Referring specifically to Fig. 3, the binders 20 are conjugated with the capture materials 50 to form conjugates C and the recognition materials 30 bind to the binders 20 whose structural conformational change has been caused by the addition of the ligand solution, as in the first embodiment described above. Here, the conjugates C of the binders 20 with the capture materials 50 may be added or the binders 20 and the capture materials 50 may be individually added to subsequently form the conjugates C. In one embodiment, the recognition materials 30 are immobilized on the surface of the immobilization members 40 and the ligand solution containing the binder-capturing material conjugates C dissolved therein is added to the immobilization members 40 to allow the recognition materials 30 to bind to the conjugates C.

Referring to Fig. 4, the recognition material-capturing material conjugates C are formed and the binders 20 whose structural conformation has been changed by adding the ligand solution bind to the recognition materials 30 of the conjugates C, as in the second embodiment described above. Also in this embodiment, the conjugates C of the recognition materials 30 with the capture materials 50 may be added or the recognition materials 30 and the capture materials 50 may be individually added to subsequently form the conjugates C. As an example, the binders 20 are immobilized on the surface of the immobilization members 40 and the ligand solution containing the recognition material-capturing material conjugates C dissolved therein is added to the immobilization members 40 to allow the binders 20 to bind to the conjugates C.

Next, a sample solution containing targets 1 of a target subpopulation is added (S200). At this time, the capture materials 50 of the conjugates C specifically bind to the targets 1. The sample solution may include a body fluid. In this case, the targets 1 are isolated from heterogeneous bulk populations in the body fluid. The bulk populations may include at least two substances selected from the group consisting of cells, exosomes, DNAs, RNAs, proteins, lipids, sugars, and metabolites used as biomarkers to diagnose target diseases. For example, exosomes as the targets 1 may be isolated from heterogeneous bulk populations including the exosomes.

Thereafter, a recovery solution is added (S300). The recovery solution is a solution that does not contain the ligands 10. When the recovery solution is added, the ligands 10 bound to the binders 20 are detached and, as a result, the changed conformation of the binders 20 is restored to their original state, leading to the dissociation of the binders 20 from the recognition materials 30. Consequently, the conjugates C bound to the targets 1 are dissociated from the recognition materials 30 (see the first embodiment and Fig. 3) or the binders 20 (see the second embodiment and Fig. 4) to recover the targets 1. At this time, the targets 1 are recovered together with the conjugates C and the recognition materials 30 (see the first embodiment and Fig. 3) or the binders 20 (see the second embodiment and Fig. 4) remain immobilized onto the immobilization members 40. Accordingly, the immobilization members 40 immobilized with the recognition materials 30 or the binders 20 can be reused to isolate targets from a new sample solution.

After addition of the sample solution, the targets may be enriched before addition of the recovery solution. Specifically, the immobilization members 40 bound to the targets 1 are captured within predetermined spaces of a solution of the ligand mixed with the sample using magnetic force, gravitational force, and/or centrifugal force. Then, the targets 1 can be enriched by removing a portion of the mixed solution where the immobilization members are absent.

This procedure may be carried out in a reactor. The immobilization members 40 immobilized with the recognition materials 30 or the binders 20 are filled in the reactor and the ligand solution, the conjugates C, and the sample solution are added thereto. The reactor may be a chromatography column where association and dissociation reactions are performed in a chromatographic manner. Alternatively, the reactor may be a batch vessel where reactions are performed in a batch manner. The targets can be isolated using magnetic force, gravitational force or centrifugal force in the reactor.

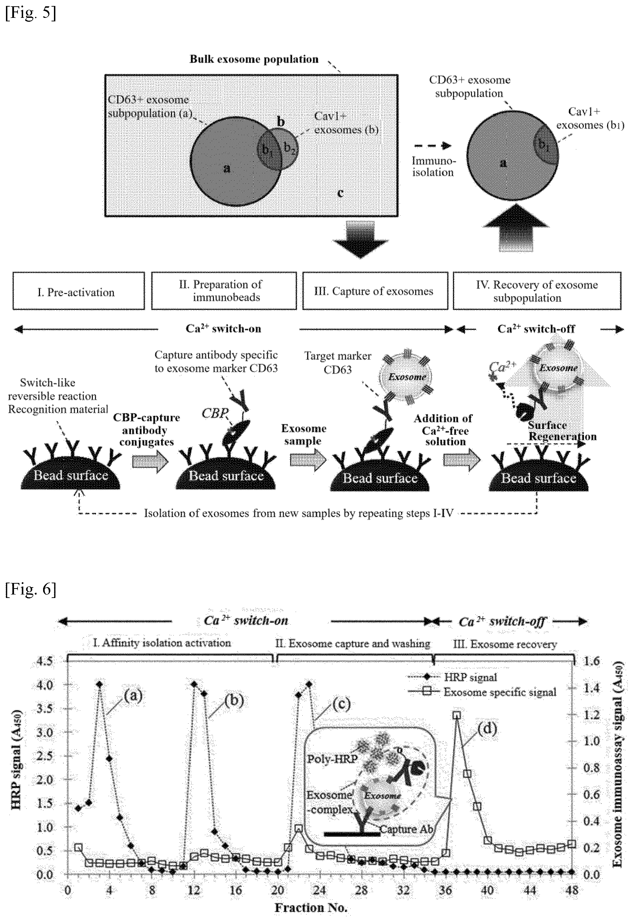

As an example, the present invention can be utilized to isolate and concentrate an exosome subpopulation from a body fluid during liquid biopsy for diagnosing a specific disease. A process for the isolation of an exosome subpopulation will be described with reference to Fig. 5. Fig. 5 is a flow diagram illustrating a process for the isolation of an exosome subpopulation associated with melanoma cancer cells using an affinity isolation method based on switch-like binding according to an embodiment of the present invention.

The conversion of exosome samples in the form of bulk populations prepared by simply concentrating body fluids to disease-relevant exosome samples selectively isolated from body fluids can lead to an improvement in the diagnostic accuracy of liquid biopsy. To experimentally exemplify this approach, melanoma cancer, a skin cancer arising from melanocytes, is selected as a target disease. For reference, melanoma cancer has the highest degree of malignancy and a high metastasis rate. Thus, melanoma cancer is a disease that requires an early, accurate diagnosis to increase the survival rate. According to a previous study, Caveolin 1 (Cavl), an exosomal surface protein, is significantly increased in the blood of patients with melanoma cancer compared to in the blood of healthy subjects. The result showed that the Cavl-positive (Cav1+) exosome-based diagnostic sensitivity is 68%, which is higher than the diagnostic sensitivity (43%) based on CD63 as another melanoma cancer marker. The clinical use of liquid biopsy with improved performance requires selective isolation and enrichment of target exosomes such as Cav1+ exosomes which, however, is difficult to achieve using general processes known in the art because of a very low proportion of the target exosomes in total exosomes from body fluids.

Therefore, the present invention intends to provide a method for diagnosing, for example, melanoma cancer with high accuracy and sensitivity using, as a test sample, a subpopulation containing Cav1+ exosomes selectively isolated from a body fluid. In consideration of the yield and reproducibility of exosome isolation, Cav1+ exosomes are not used as isolation targets but a population of exosomes associated with the disease is used as a primary isolation target. For example, since CD63 protein, a house-keeping protein abundantly found in general exosomes, is increased and expressed on the surface of exosomes when certain cancers (including melanoma cancer) occur, compared to other surface proteins, this surface protein can be used as an initial isolation mediator. In practice, the sensitivity of the exosomal surface protein CD63+ exosomes as diagnostic markers for melanoma cancer is 43%, which is reportedly lower than that of the exosomal surface protein Cav1+ exosomes. It is, however, assumed that the sensitivity of a CD63+ exosome subpopulation containing Cav1+ exposomes as a test sample in the diagnosis of melanoma cancer is significantly high compared to that of a bulk population sample (see the top of Fig. 5 for the definition of terms).

An immunoisolation technique using antibodies specific to target markers can be applied to the isolation of exosomes according to their surface protein markers. Utilizing this technique, target exosomes can be isolated by reacting an exosome sample with the specific antibodies immobilized on solid surfaces such as magnetic beads or gel beads and allowing the exosomes to unbind from the antibodies. This technique is more suitable for isolating exosomes associated with surface proteins than other existing isolation techniques (*e.g*., ultracentrifugation, accelerated sedimentation, and size-based isolation) due to the high selectivity intrinsically offered by the antigen-antibody reaction. However, the use of harsh conditions such as acidic pH is required to recover the exosomes bound to the antibodies via the antigen-antibody reaction from the solid surfaces but may cause damage to the exosomes and make it difficult to recover the exosomes in high yield. In contrast, the present invention uses a new binding reaction such as a reversible antigen-antibody reaction to separate and concentrate an exosome subpopulation including target markers with high efficiency under mild conditions.

According to one embodiment of the present invention, reversible recognition materials are immobilized on solid surfaces (*e.g*., bead surfaces) (I in Fig. 5, the bottom) and calcium binding proteins (CBPs) are conjugated with antibodies specific to exosomal surface proteins (*e.g*., CD63) via biotin-streptavidin linkages to form CBP-capture antibody conjugates. The CBP-capture antibody conjugates are dissolved in a solution containing calcium ions (*e.g.,* >10 mM Ca²⁺) and are added to the immobilized reversible recognition materials. The conjugates are immobilized on the solid surfaces by a "switch-on" recognition reaction (II in Fig. 5). When a bulk exosome sample is prepared and added to the solution, the exosomes having CD63 markers react with and are captured by the capture antibodies on the solid surfaces (III in Fig. 5). After washing with the same solution, a calcium-free exosome recovery solution is added. The calcium ions are detached from the CBPs, resulting in a "switch-off' state. Thus, the captured exosomes bound to the capture antibodies can be recovered into the solution (IV in Fig. 5). As a consequence, a subpopulation of the CD63+ exosomes is isolated and recovered. The exosomes can be enriched according to the volume ratio between the sample solution and the recovery solution used. In addition, the solid surfaces can be regenerated and reused for the separation of other samples.

The present invention will be more specifically explained with reference to the following examples, including evaluation examples. However, these examples are provided for illustrative purposes only and are not intended to limit the scope of the invention.

### Materials

The present inventors have produced monoclonal antibodies specifically recognizing the unique structure of altered calcium binding proteins (CBPs) bound with calcium ions (Paek et al. Analyst 139, 3781-3789, 2014). Mutated glucose-galactose binding protein (GGBPs) were selected as the CBPs. These proteins were produced from *Escherichia coli* (*E. coli*) and purified. Sodium chloride, trizma, casein (sodium salt form, extract from bovine milk), 3,3',5,5'-tetramethylbenzidine (TMB), sodium dodecyl sulfate (SDS), bovine serum albumin (BSA), and CNBr-activated Sepharose 4B gel (4% agarose) were purchased from Sigma (St. Louis, MO, USA). Calcium chloride dihydrate and potassium chloride were supplied by Daejung (Siheung, Korea). Sulfosuccinimidyl-6-[biotinamido]-6-hexanamido hexanoate (NHS-LC-LC-biotin), succinimidyl 4-(N-maleimidomethyl)cyclohexane-1-carboxylate (SMCC), N-succinimidyl 3-(2-pyridyldithio)-propionate (SPDP), dithiothreitol (DTT), maleimide, streptavidin (SA), and horseradish peroxidase (HRP) were purchased from Thermo Fisher Scientific (Rockford, IL, USA). Anti-CD63 antibody (Cat. No. 353014) was supplied by BioLegend (San Diego, CA, USA). Monoclonal antibodies specific for CD9 (Cat. No. MAB1880), CD81 (Cat. No. MAB4615) or Caveolin-1 (Cavl; Cat. No. MAB5736) were supplied by R&D Systems (Minneapolis, MN, USA). Lyophilized exosome standard reagents (human plasma-derived) and melanoma cancer cell cultures from healthy subjects were purchased from HansaBioMed (Tallinn, Estonia). Magnetic beads (Dynabeads M-280 Tosylactivated, 2.8 mm diameter) and streptavidin-poly-HRP20 were supplied by Invitrogen (Carlsbad, CA, USA) and Fitzgerald (Acton, MA, USA), respectively. Several other exosome isolation kits, including ExoQuick^{™} kit (System Biosciences; Mountain View, CA, USA), MagCapture^{™} kit (Wako; Osaka, Japan), and exoEasy Maxi^{™} kit (Qiagen; Hilden, Germany), were purchased from different sources. Other reagents used were of analytical grade.

### Example 1: Preparation of components for exosome isolation and characterization

***Conjugation between SA and CBP.*** CBP (400 µg) was dissolved in a 10 mM phosphate buffer (pH 7.4; PB) and mixed with 20-fold molar excess of SMCC. The mixture was allowed to react at room temperature for 1 h to activate CBP. SA (2 mg) dissolved in PB was reacted with 5-fold molar excess of SPDP for 1 h and chemically reduced with 10 mM DTT solution containing 5 mM ethylenediaminetetraacetic acid (EDTA) at 37 °C for 1 h to expose sulfhydryl groups. The activated components were separated on a Sephadex G-15 gel filtration column (10 ml volume) to remove excess reagents. The two activated proteins (*i.e.* maleimide-CBP and thiolated-SA) were mixed in a 1:5 molar ratio. The mixture was allowed to react at room temperature for 4 h. Thereafter, the final solution containing the CBP-SA polymer was stored at 4 °C in a tightly-sealed container before use.

***Biotinylation of anti-CD63 antibody.*** The succinimidyl-ester moiety of NHS-LC-LC-biotin was reacted with the amine group on the antibody molecule to biotinylate the antibody. Briefly, anti-CD63 antibody dissolved in PB solution containing 140 mM NaCl (PBS) was mixed with NHS-LC-LC-biotin dissolved in 20-fold molar excess of dimethyl sulfoxide (DMSO). The mixture was allowed to react at room temperature for 2 h. Unreacted reagents in the reaction mixture were removed by size-exclusion chromatography using a Sephadex G-15 column equilibrated with PBS. The purified conjugate was dialyzed twice against PBS, concentrated to 1 mg/ml with Vivaspin 500, and stored at 4 °C before use.

***Labeling of anti-CD63 antibody with HRP.*** Anti-CD63 antibody was labeled with HRP according to the general procedure. Briefly, first, the antibody was reduced with 10 mM DTT at 37 °C for 1 h and excess reagent was removed with a gel filtration column. HRP was activated with 30-fold molar excess of SMCC and unreacted reagent was removed by size-exclusion chromatography as before. After the reduced antibody was mixed with 10-fold molar excess of the activated HRP, conjugation was performed at 4 °C for ≥ 24 h. The resulting conjugate was mixed with an equal volume of glycerol and stored at -20 °C.

### Example 2: Immunoaffinity chromatography

***Preparation of immunoaffinity gel.*** For immunoaffinity chromatography, the CBP conformation-specific monoclonal antibody was immobilized on the surface of the CNBr-activated Sepharose 4B gel. Lysine residues on the antibody molecule were reacted with functional groups on the gel under alkaline conditions to chemically bind the antibody (2 mg per ml of the hydrated gel) to the agarose gel according to the manufacturer's instructions. After antibody binding, the remaining reactive groups on the gel were hydrolyzed with Tris-HCl buffer.

The resulting immunoaffinity gel was packed in a plastic column to fabricate an immunoaffinity chromatography column, which was then washed under alternating acidic and alkaline conditions to remove the antibody non-specifically adsorbed to the solid (gel) surface.

***Fabrication of immunoaffinity chromatography column and isolation of CD63 positive (CD63*+*****) exosomes through the column.*** The immunoaffinity gel was used to fabricate an immunoaffinity chromatography column. An immunoaffinity chromatography column specific to CD63-positive (CD63+) exosomes was fabricated by the following procedure. First, a plastic column was packed with the immunoaffinity gel. The immunoaffinity gel was immobilized with the CBP-SA conjugate (5 µg/ml) by reacting with the CBP conformation-specific monoclonal antibody using a transport solution containing Ca²⁺ (10 mM) ("Ca²⁺ switch-on" condition). Thereafter, the biotinylated anti-CD63 antibody (3 µg/ml) was immobilized on the CBP-SA conjugate layer via biotin-SA binding.

The fabricated immunoaffinity column (9 mm × 3.2 mm; 1.5 ml bed volume) was used for exosome isolation. Specifically, a standard exosome sample from a healthy subject (25 µg/ml; 1 ml) was loaded onto the CD63 immunoaffinity column. A Ca²⁺-containing transport solution was injected to wash off unbound components. Thereafter, a Ca²⁺-free solution ("Ca²⁺ switch-off condition) was injected to recover fractions of the CBP-antibody-exosome complex (containing CD63+ exosomes) bound to the immunoaffinity gel. Here, the immunoaffinity column was regenerated and reused for analysis of other samples.

More specifically, the gel was initially equilibrated with 20 mM Tris-HCl containing 140 mM NaCl, 10 mM Ca²⁺, and 2% BSA (pH 7.4; binding solution) before addition of an exosome sample to the immunoaffinity column. Each of the CBP-SA (5 µg/ml, 1 ml) and the biotinylated anti-CD63 antibody (3 µg/ml, 1 ml) prepared in Example 1 and an exosome standard sample (1 ml; normal exosome standard reagent or melanoma cancer cell culture) was diluted with a binding solution. The HRP enzyme (0.1 µg/ml) was added to each dilution for marking the addition position of each reagent. The resulting solutions were sequentially added to the gel column such that they were eluted at predetermined elution volumes. The eluate from the column was transferred to a fraction collector (fraction volume: 1 ml) using a peristaltic pump at an elution rate of 120 µl/min. Unreacted proteins were removed from the gel column by washing with a BSA-free binding solution. Finally, a 20 mM Tris-HCl buffer solution (pH 7.4) containing 500 mM NaCl was fed into the column to elute and recover exosomes bound to the column. Thereafter, a 100 mM sodium acetate buffer (pH 4.5) containing 500 mM NaCl, 5 % (v/v) Tween-20, and 0.02 % (w/v) thimerosal was fed into the gel column for regeneration, and the column was thoroughly washed until no protein elution was observed.

### Example 3. Immunomagnetic isolation

***Immunomagnetic enrichment of CD63*+** ***exosomes.*** Generally, magnetic enrichment is a method for separating and concentrating a target substance through an antigen-antibody reaction using antibody-bound immunomagnetic beads under a magnetic field. The isolation of CD63+ exosomes based on this method is basically the same as the immunoaffinity chromatography performed in Example 2. The magnetic beads were bound with the monoclonal antibody recognizing a conformational change of CBP by Ca²⁺ binding, followed by sequential reaction with the CBP-SA conjugate and the biotinylated anti-CD63 antibody under the "Ca²⁺ switch-on" condition. The resulting immunomagnetic beads were allowed to react with an exosome sample (normal exosome standard sample (25 µg/ml; 1 ml)). The magnetic beads were captured with a magnet, the supernatant was removed, and the beads were washed. Then, the buffer solution was added under the "Ca²⁺ switch-off' condition to recover and concentrate CD63+ exosomes. The volume of the buffer solution used was one-tenth of its initial volume.

More specifically, for magnetic enrichment, the amine groups on the CD63-specific antibody were chemically bound to and immobilized onto the activated tosyl moieties on the solid surfaces of the magnetic beads. After washing 3 times with Tris-HCl buffer, the remaining surfaces were blocked with casein-PBS. After magnetic separation of the beads (total 1 mg) from the solution, the CBP-SA conjugate (5 µg/ml; 1 ml) diluted with a binding solution was added and incubated on a shaker for 1 h. After washing the beads with a BSA-free binding solution, the biotinylated anti-CD63 antibody (3 µg/ml; 1 ml) was incubated under the same conditions. After washing again, an exosome sample (25 µg/ml; 1 ml) diluted with a binding solution was added and allowed to react for 3 h. Finally, CD63+ exosomes were recovered and the beads were regenerated according to the same protocol as in immunoaffinity chromatography.

### Evaluation Example 1: Analysis of the eluent fractions recovered in Example 2

Fig. 6 shows the results of immunoaffinity chromatography during isolation of CD63+ exosomes from the sample using the inventive affinity isolation system and method based on switch-like binding.

First, a chromogenic enzyme assay for horseradish peroxidase (HRP) was conducted to determine the elution positions of the reagents added in Example 2 on the chromatogram. Analysis of the eluent fractions collected from the column during elution in Example 2 confirmed the addition position of each component and the distribution of CD63+ exosomes in each fraction. When each of the components (*i.e.* the CBP-SA conjugate, the biotinylated anti-CD63 antibody, and the exosome sample) was injected into the pretreated gel column, each solution was allowed to contain a total of 0.1 µg of HRP such that the addition position after elution was determined. To this end, portions of each fraction were sampled and plated in microwells and an HRP substrate solution was added thereto. From the results, a chromatogram for information on the injection locations of the components was obtained. Peaks (a), (b), and (c) are shown by dotted lines in Fig. 6. More specifically, for the enzyme assay, a predetermined amount (5 µl) of each fraction was plated in a microtiter plate and then an enzyme substrate solution (200 µl) was added to generate a signal from the enzyme. The enzyme substrate solution was prepared by mixing a 3% (v/v) aqueous hydrogen peroxide solution (10 µl), 10 mg/ml TMB (100 µl) dissolved in dimethyl sulfoxide (DMSO), and a 0.05 M acetate buffer (pH 5.1; 10 ml). The reaction was stopped with the addition of a 2 M sulfuric acid solution (50 µl) before saturation of the enzyme signal. The optical density was measured with a microplate reader (Synergy H4, BioTek Inc.; Winooski, VT, U.S.A.) at a maximum absorbance of 450 nm.

Furthermore, enzyme-linked immunosorbent assay (ELISA) was conducted on exosomes in each fraction to determine the elution position of the exosomes isolated and recovered via immunoaffinity chromatography. To this end, portions of each fraction were sampled and plated in microwells immobilized with the anti-CD63 antibody, followed by incubation. Thereafter, the SA-poly HRP 20 conjugate was allowed to react with the remaining biotin on the biotinylated antibody molecules predicted to react with exosomes in the column. More specifically, 5 µg/ml antibody (100 µl) was added to each well and incubated at 37 °C for 1 h for antibody immobilization. After washing, the remaining surfaces were blocked with Tris-HCl buffer containing 0.5% casein (casein-Tris). Then, an aliquot (100 µl) of each fraction was added to each well and incubated in a container maintained at 37 °C and a humidity of 100% for 12 h. The wells were washed and the biotinylated anti-CD63 antibody (1 µg/ml; 100 µl) prepared in Example 1 was added thereto, followed by incubation at 37 °C for 1 h. After washing again, SA-poly HRP20 (66 ng/ml; 100 µl per well) diluted with casein-Tris was added and incubated at 37 °C for 1 h. Finally, a substrate solution for HRP (200 µl) was added and the reaction was stopped with the addition of a 2 M sulfuric acid solution (50 µl) before color development was saturated. The optical density was measured as above. The exosome-specific chromatography results obtained by adding the HRP substrate solution are shown by solid lines in Fig. 6. Significant signals under the "Ca²⁺ switch-off condition are indicated by a peak (d) in Fig. 6. Since only the anti-CD63 antibody sandwich complexes formed around the CD63+ exosomes were detected by the immunoassay (see the inset in Fig. 6), the peak (d) eluted from the column was determined to contain CD63+ exosomes separated and recovered by immunoisolation. In addition, the generation of weak signals even at the injection location of the exosome sample (peak (c) in Fig. 6) was predicted as a result of elution of some exosome complexes or non-specific reaction of excess exosomes having no affinity for the column. However, the peak (c) was excluded from further analysis because its size was less than 10% of that of the peak (d).

### Evaluation Example 2: Evaluation of reproducibility of the immunoaffinity chromatography of Example 2

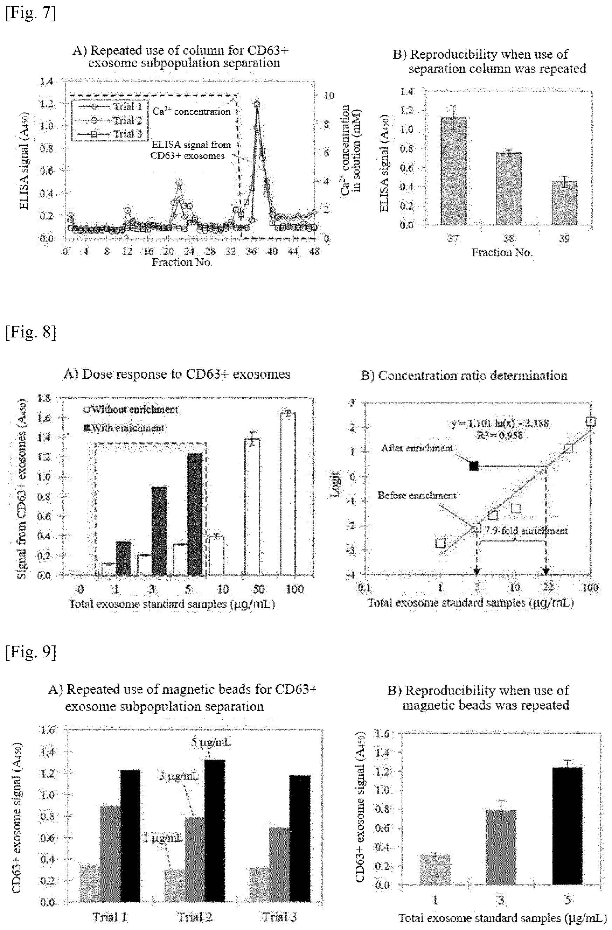

Fig. 7 shows the reproducibility of exosome isolation when the immunoaffinity chromatography of Fig. 6 was repeated.

To confirm the reproducibility of exosome isolation using the immunoaffinity column of Example 2, the same immunoisolation procedure was repeated three times. As already mentioned above, eluent fractions obtained from each experiment were subjected to immunoassay on CD63+ exosomes to obtain their chromatograms (see Fig. 7, A). After exchange with a Ca²⁺-free transport solution for elution (after the 35^{th} fraction), CD63+ exosomes were isolated and formed single independent peaks despite repeated experiments. Moreover, the fraction numbers forming the CD63+ exosome peaks and the intensities of specific signals from exosomes were found to be very similar. Particularly, the immunoassay signals from CD63+ exosomes present in the fraction Nos. 37, 38, and 39 in the recovered exosome peaks on the chromatogram showed an average coefficient of variation (CV) of ≤ 9.4%, indicating high reproducibility (Fig. 7, B).

More specifically, first, an immunoaffinity chromatogram was obtained as above and exosomes present in each eluent fraction were recovered and analyzed according to the same isolation protocol. This procedure was repeated three times. The results of immunoassay for CD63+ exosomes present in the main fractions constituting each peak for isolated exosomes were obtained. The coefficient of variation (CV) between the measured signals was used as an index for the evaluation of reproducibility for isolation tests. The gel column for isolation was filled with Tris-HCl buffer and stored at 4 °C when not in use.

### Evaluation Example 3: Evaluation of enrichment performance of Example 3

Fig. 8 shows the results of immunomagnetic isolation during isolation and enrichment of CD63+ exosomes from the sample using the inventive affinity isolation system and method based on switch-like binding.

To test the enrichment performance of Example 3, magnetic isolation was repeatedly applied to other standard exosome standard samples (1, 3 or 5 µg/ml), followed by sandwich enzyme-linked immunosorbent assay on CD63+ exosomes, as described above. The results are shown as black bars in Fig. 8, A. The results of immunoassay for CD63+ exosomes in the original standard samples before magnetic enrichment are shown in white bars in Fig. 8, A. Comparing the results of the samples before and after magnetic isolation, the intensities of signals generated by magnetic enrichment increased by an average of about 3.7 times. The dose responses of the immunoassay for the original standard samples were linearized by log-logit transformation to determine the enrichment ratio of the isolated exosomes and the concentrations of the enriched CD63+ exosomes were determined therefrom (Fig. 8, B). Particularly, the 3 µg/ml exosome sample was enriched to 22 µg/ml based on CD63+ exosomes, which corresponds to an enrichment efficiency of approximately 7.9 times.

The concentrations of the exosome samples before and after isolation were determined by comparison with the results obtained with the standard reagent for immunoassay. First, the normal exosome standard reagent was diluted with casein-Tris to prepare exosome standard samples (1-100 µg/ ml). Anti-CD63 antibody (5 µg/ml; 100 µl) was plated in microwells for immunoassay, incubated at 37 °C for 1 h, and immobilized on the inner surfaces of the wells with a capture binder. After washing, the remaining surfaces were blocked with casein-Tris under the same conditions as above. The exosome samples (each 100 µl) were plated in different wells and incubated at 37 °C for 12 h. The subsequent procedure was performed according to the ELISA protocol described above.

### Evaluation Example 4: Evaluation of reproducibility of the immunomagnetic isolation of Example 3

Fig. 9 shows the reproducibility of exosome isolation when the immunomagnetic isolation of Fig. 8 was repeated.

The magnetic enrichment technique based on the switch recognition material in Example 3 enables capture of target exosomes under the "Ca²⁺ switch-on" condition, sequential recovery of exosomes under the "Ca²⁺ switch-off condition, regeneration of pretreated magnetic beads, and reuse of pretreated beads for isolation and enrichment of new samples. To evaluate the reproducibility of enrichment performance for actual reuse of pretreated beads, different concentrations of exosome standard samples (1, 3, and 5 µg/ml; collected from normal human plasma) were magnetically isolated and enriched and the same procedure was repeated twice using the reused pretreated beads. The concentrations of the isolated exosome samples were analyzed by sandwich enzyme-linked immunosorbent assay for CD63+ exosomes. The results are shown in Fig. 9, A. Referring to Fig. 9, A, the concentration response patterns for CD63+ exosomes were very similar when the samples were isolated and enriched using the pretreated beads. In practice, the enrichment of CD63+ exosomes in the isolated samples depending on the exosome concentration was found to be very reproducible with an average CV of 8.3% based on the signal values from immunoassay (see Fig. 9, B).

Here, the standard samples (1, 3, and 5 µg/ml) were prepared by diluting an exosome standard reagent from a healthy subject with a binding solution. The magnetic beads were immersed in casein-Tris and stored at 4 °C when not in use.

### Evaluation Example 5: Characterization of CD63+ exosomes isolated in Examples 2 and 3

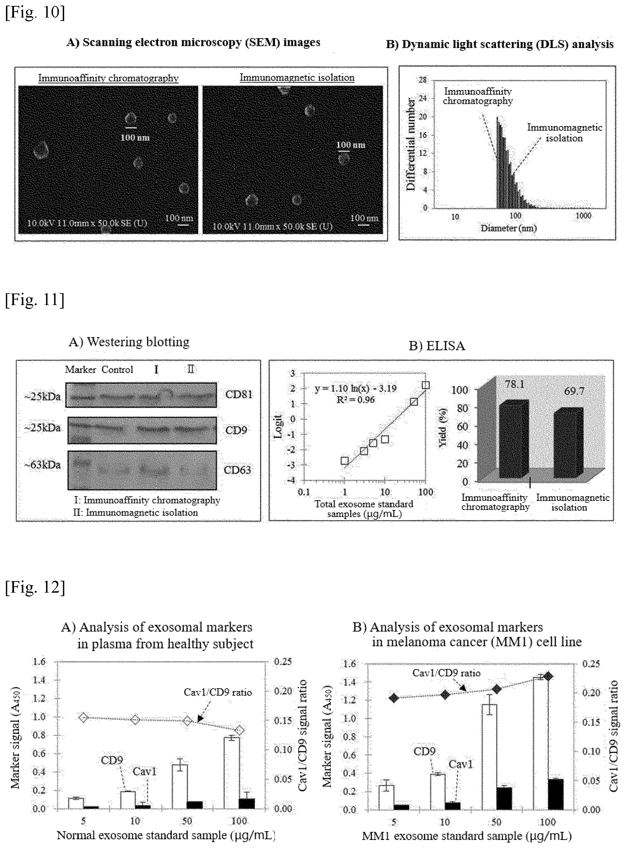

Fig. 10 shows the results of physical characterization for CD63+ exosomes isolated using the inventive affinity isolation system and method based on switch-like binding. Fig. 11 shows the results of immunochemical characterization for exosome surface markers of CD63+ exosome samples isolated using the inventive affinity isolation system and method based on switch-like binding.

Two characteristics (including morphology of microvesicles and distribution of different types of surface proteins) of exosomes were actually determined through morphological characterization of the CD63+ exosome subpopulations separated in Examples 2 and 3 and immunoassay for surface proteins. For morphological characterization, the morphology of exosomes isolated by the inventive method was measured. To this end, images were observed using a scanning electron microscope (SEM) and the size distribution was measured by dynamic light scattering (DLS). The distribution of exosomal proteins was determined by Western blotting. The recovery rate after isolation was measured by enzyme-linked immunosorbent assay (ELISA).

***Scanning electron microscopy (SEM) imaging.*** The morphologies of the CD63+ exosome samples isolated in Examples 2 and 3 were observed by SEM imaging. To obtain high magnification images, specimens were produced from the exosome samples separated by immunoaffinity chromatography or immunomagnetic isolation according to the standard protocol. To measure the solid-state microstructure and morphology, each original sample was transferred to a microscope glass slide and covered with a coverslip, and the object was immersed in liquid nitrogen and fixed by quick freezing. After removal of the coverslip, the frozen specimen was fixed by the addition of a 2.5% glutaraldehyde solution, washed with Tris-HCl buffer and deionized water (each once), and dehydrated with acetone. Then, acetone was removed using critical-point drying with liquid carbon dioxide. The samples were mounted on aluminum stubs using a tempfix mounting adhesive, and their surfaces were brought into contact with colloidal silver. After platinum was sputter-coated to a thickness of 3-5 nm, the samples were observed with a Hitachi S-4700 SEM (Hitachi; Tokyo, Japan). In the image of each sample, spherical particles with a diameter of ∼ 100 nm were observed (see Fig. 10, A). In practice, exosomes are membrane microvesicles released from various types of cells and are known to have a diameter of approximately 30-200 nm.

***Dynamic light scattering (DLS) analysis.*** To more accurately measure the size distribution of each separated CD63+ sample, DLS analysis was conducted on exosomes using a particle size analyzer (ELSZ-1000, Otsuka Electronics; Osaka, Japan) at room temperature. The exosome sample (0.5 ml) was transferred to a disposable cuvette (BI-SCP, Brookhaven Instruments Corporation; NY, USA) and analyzed according to the manufacturer's guidelines. After the size of exosomes was determined according to the Stokes-Einstein equation, the size distribution of extracellular vesicles was characterized. As a result, the average particle diameter in each sample was found to be at the level of -186.2 nm or ∼193.8 nm (see Fig. 10, B). From the measured particle size and particle size distribution, it is believed that the particles present in the sample separated by immunoaffinity chromatography and immunomagnetic isolation are exosomes.

***Western blotting.*** To determine the distribution of different types of surface proteins, which is one of the general characteristics of exosomes, Western blotting was conducted on tetraspanin protein markers in the CD63+ exosome samples separated in Examples 2 and 3. After SDS-PAGE analysis was conducted on the exosome samples under optimized conditions, the isolated proteins were transferred to a nitrocellulose membrane and antibody-HRP conjugates specific for CD9, CD63, and CD81, which are usually abundantly distributed on the exosome surface, were allowed to react with the respective target proteins. More specifically, first, each exosome sample was treated with a 50 mM Tris buffer (pH 8.0) containing 150 mM NaCl, 1% Triton X-100, 0.5% sodium deoxycholate, and 0.1% sodium dodecyl sulfate (SDS). The denatured proteins were isolated using 8% SDS-polyacrylamide gel electrophoresis (SDS-PAGE) and transferred onto a nitrocellulose membrane by electrophoresis. After the remaining surfaces were blocked with casein-PBS, blotting was conducted using anti-CD63, CD9, and CD81 antibodies, which had previously been chemically conjugated with HRP. Finally, a HRP substrate solution containing 3,3'-diaminobenzidine (DAB) as a chromogenic reagent was added to generate colorimetric signals on the membrane. The substrate solution was prepared by adding 0.05% DAB (10 µl) and 3% H₂O₂ (1 µl) to a 50 mM sodium acetate buffer (pH 5.0; 1 ml).

The stained colorimetric signals demonstrated that all tetraspanin protein markers of exosomes were expressed in the CD63+ exosome samples separated in Examples 2 and 3 (see Fig. 11, A). For reference, the band signals from CD63 were not clear compared to the colorimetric signals of the bands for CD9 and CD81. This was affected by the relatively wide distribution of the bands (30-60 kDa) because the CD63 markers are glycosylated proteins, but the more fundamental reason was that the band signal for the expression of CD63 markers in the exosome standard sample (Fig. 11, A; Control) used in this evaluation experiment was low (see Fig. 11, A). Taken the above results together, the samples separated by immunoaffinity chromatography and immunomagnetic isolation exhibit all general characteristics of exosomes, indicating that CD63+ exosome subpopulations were successfully separated by the inventive method.

***Recovery rate of CD63*+** ***exosomes after isolation.*** To determine the recovery rates of exosome isolation systems, CD63+ exosomes in the samples were quantitatively analyzed before and after isolation and the concentrations of the CD63+ exosome subpopulations were indirectly measured using a standard curve obtained through immunoassay on CD63 markers for the exosome standard samples. The standard curve was obtained by enzyme-linked immunosorbent assay (ELISA) using an antibody specific to CD63, an exosome surface marker, as a capture and detection binder during immunoassay (Fig. 11, B; left). Immunoassay was conducted on the samples under the same conditions before and after isolation. The resulting signals were substituted into the standard curve to calculate the concentrations of exosomes. After calibration of the volume dilution factor, the total amounts of exosomes in the exosome samples before and after immunoaffinity chromatography and immunomagnetic isolation were calculated to determine the recovery yields of the isolation processes (Fig. 11, B; right). The yields of immunoaffinity chromatography and immunomagnetic isolation were found to be ∼78.3% and ∼68.5%, respectively, which were very high compared to the yields of conventional isolation processes (*e.g*., < 50%) for recovering isolation products under severe conditions such as acidic pH. This is because the affinity of the recognition material for CBP was lowered to the background level when calcium ions were removed from the solution in the isolation process using the switch recognition material, resulting in relatively high recovery yield of the isolated exosomes and minimal structural deformation of exosomes.

### Evaluation Example 6: Evaluation of analysis performance when CD63+ exosome subpopulations were used as diagnostic samples

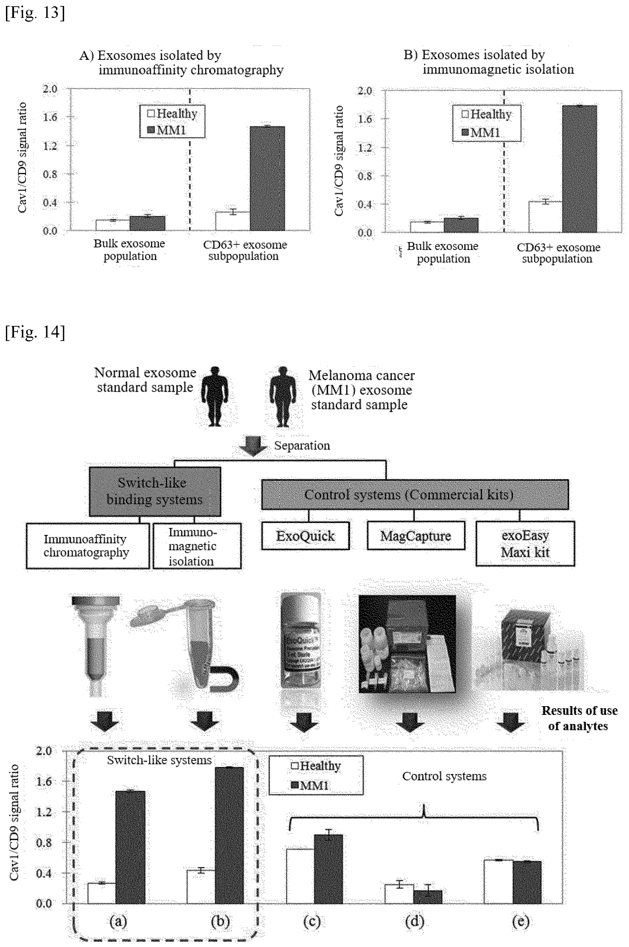

Fig. 12 shows the corrected concentrations of Cavl based on the concentration of CD9 to compensate for variations in concentration between exosome standard samples. Fig. 13 shows the potential effects of the use of CD63+ exosomes isolated using the inventive affinity isolation system and method based on switch-like binding reaction as analytical samples on melanoma cancer diagnosis.

***Concentration normalization of target markers.*** When the separated CD63+ exosome subpopulations were used as diagnostic samples, high-sensitivity diagnosis is predicted due to the enrichment effect of Cav1+ exosomes as melanoma cancer-associated markers. However, when the expression level of Cavl protein is simply measured, there is a limitation in accurately determining the isolation effect due to the different concentrations of exosomes in the samples. Hence, a method for normalizing the marker through calibration based on CD9 normally present on the surface of exosomes was introduced to minimize uncertainty during performance comparison. To this end, Cavl protein-specific antibodies and CD9 protein-specific antibodies were immobilized onto different wells of a microtiter plate, the same sample was plated in the wells, followed by incubation. ELISA was performed using CD63 antibodies as detection antibodies. As a result, signals proportional to the concentrations of the cancer markers and the normal markers were obtained (see the bars in Fig. 12) and the ratios of the signals (that is, Cav1/CD9 signal ratios) were calculated (see the line in Fig. 12). When a plasma sample from a healthy subject was diluted and analyzed (Fig. 12, A), the Cav1/CD9 signal ratios were, on average, 0.147, which was maintained almost constant at different dilution factors. For melanoma cancer cell samples (Fig. 12, B), relatively high signals were measured for the markers and the Cav1/CD9 ratios were, on average, 0.206, indicating that the Cavl expression levels were ∼40% higher on average than those of the standard samples. As such, changes in total exosome concentration could be normalized by calibrating Cav1+ exosomes in the CD63+ exosome subpopulations with CD9+ exosomes as normal markers.

To elaborate on the evaluation experiment procedure, the exosome standard samples (5-100 µg/ml) were prepared by diluting exosomes obtained from normal plasma or a melanoma cancer cell line with casein-Tris. The concentrations of markers in the samples were measured as follows. First, capture antibodies (5 µg/ml; 100 µl) specific for the markers were plated in microwells and incubated at 37 °C for 1 h for immobilization. The subsequent procedure was performed in the same manner as in the ELISA procedure for CD63 described above. Signals generated after analysis for CD9 and Cavl were measured and the Cav1/CD9 signal ratios were calculated for the samples.

***Potential of separated exosome subpopulations as diagnostic samples.*** The potential effects of the CD63+ exosome subpopulations as diagnostic samples separated based on switch recognition materials on the diagnosis of melanoma cancer were tested using the method for measuring the Cav1/CD9 signal ratio by immunoassay. Healthy exosome and melanoma cancer exosome standards (each 25 µg/ml) were separated to obtain CD63+ exosome subpopulations and the expression levels of Cavl protein were compared with those before separation. The Cav1/CD9 signal ratio of the bulk cancer exosome sample before separation was increased by ∼40% compared to that of the bulk healthy exosome sample before separation (Fig. 13, A; left). The signal ratio of the cancer CD63+ exosome subpopulation sample separated by immunochromatography was increased by ∼4-fold (*i.e.* 400%) compared to that of the healthy CD63+ exosome subpopulation sample separated by immunochromatography (Fig. 13, A; right). That is, the use of the separated CD63+ exosome subpopulation sample resulted in a ∼10-fold increase in potential sensitivity of melanoma cancer cell screening. The increased potential sensitivity to melanoma cancer cell screening was also found for the use of the subpopulation sample separated by immunomagnetic isolation (Fig. 13, B). This effect is predicted to be due to the use of the CD63+ exosome subpopulation as a screening sample that leads to not only an increase in the concentration of Cav1+ exosomes but also a decrease in the concentration of CD9+ exosomes. It has indeed been reported that the concentrations of CD9+ exosomes are reduced in cancer samples.

### Evaluation Example 7: Performance comparison with conventional exosome isolation systems

Fig. 14 compares the abilities of the inventive affinity isolation systems based on switch-like binding reaction to distinguish melanoma cancer samples from normal samples based on a Cav1/CD9 concentration ratio determined by immunoassay of the separated samples with those of currently commercially available exosome isolation systems.

The effects of the CD63+ exosome subpopulation samples obtained using the switch recognition material-based isolation systems in Examples 2 and 3 on melanoma cancer cell screening were objectively evaluated by comparison with those of samples obtained using currently commercially available exosome isolation kits. The commercially available exosome isolation kits were sold under the trade names ExoQuick^{™}, MagCapture^{™}, and exoEasy Maxi^{™}. Twenty five µg/ml normal exosome and melanoma cancer exosome standards (each 1 ml) were isolated according to the established protocol of each system. The expression levels of Cavl and CD9 proteins in the isolated exosome samples were measured as signals by ELISA, as described above. The Cav1/CD9 signal ratios as calibration parameters were calculated and compared. The results are shown in Fig. 14. Referring to Fig. 14, the Cav1/CD9 signal ratios of the cancer CD63+ exosome subpopulation samples obtained by the inventive immunochromatography and immunomagnetic isolation systems were increased by 5.5-fold and 4.1-fold in a cancer sample, respectively, compared to those of the normal samples, as mentioned above. In contrast, there were no substantial differences in Cav1/CD9 signal ratio between the samples obtained using the commercially available isolation kits and the samples before separation, regardless of the kits used. Rather, the Cav1/CD9 signal ratios of the cancer samples were decreased compared to those of the normal samples.

These results are thought to be because the isolation principles of the commercial exosome isolation kits are different from the principles of the inventive isolation systems. The commercial kits are based on a precipitation method for exosome isolation using a polymer such as polyethylene glycol (ExoQuick^{™}, SBI), an affinity method for exosome isolation using a protein reacting with phosphatidylserine (PS) on the exosome surface in the presence of metal ions (MagCaprue^{™}, Wako), and a method for exosome isolation using a spin column to shorten the time required for isolation (exoEasy Maxi^{™}, Qiagen).

Most of the commercial kits are only used to separate or concentrate bulk exosome populations and are limited in significantly improving diagnostic sensitivity, making it difficult to apply to cancer cell screening. In addition, the conventional immunoisolation targeting specific exosome surface markers can be applied to increase diagnostic sensitivity but is not competitive in terms of exosome recovery yield and maintenance of intact form without introducing an innovative method using a switch recognition material as in the present invention.

Hereinafter, processes for exosome isolation using the commercial kit used in this evaluation experiment will be described briefly.

***Exosome isolation using ExoQuick kit.*** Exosome standard samples were isolated according to the manufacturer's guidelines. Briefly, commercial exosome samples (each 25 µg/ml; 1 ml) were separately transferred into microwells, and predetermined amounts of ExoQuick exosome precipitation solution were added thereto. After mixing, the mixtures were refrigerated at 4 °C. The mixtures were centrifuged and the supernatant was discarded. The exosomes precipitated in the form of granules were collected, redissolved in a buffer (500 µl), and subjected to immunoassay to determine the Cav1/CD9 ratio as described already.

***Exosome isolation using MagCapture kit.*** The same exosome standard sample was isolated according to the manufacturer's protocol. Briefly, magnetic beads (60 µl) coated with phosphatidylserine (PS)-bound protein, a specific capture factor for exosomes, were added to a sample (1 ml) containing metal ions as binding promoters. The mixture was allowed to react with stirring for 3 h. Thereafter, the beads were washed three times with a washing solution (2 mM CaCh containing 150 mM NaCl, 0.05% Tween20, and 20 mM Tris-HCl (pH 7.4; 1 ml)) and eluted with an eluent (20 mM Tris-HCl containing 150 mM NaCl and 2 mM EDTA (pH 7.4)). Immunoassay was conducted to determine the Cav1/CD9 ratio for the recovered exosome sample.

***Exosome isolation using exoEasy Maxi kit.*** An exosome sample was isolated using another commercial kit. Briefly, XBP buffer (1 ml) provided with the kit was added to an equal volume of the sample. After mixing gently, the mixture was left standing at room temperature and preheated to room temperature. The mixture was transferred into an exoEasy spin column, followed by centrifugation for 1 min. After XWP buffer solution (10 ml) was loaded onto the column, centrifugation was performed for additional 5 min to remove the loaded buffer solution. The spin column was transferred to a new recovery tube and XE buffer (500 µl) was then loaded onto the column, followed by incubation for 1 min. After centrifugation for additional 5 min, exosomes were eluted and recovered in a tube. Finally, the eluate was again loaded onto a spin column, incubated for 1 min, and centrifuged for 5 min. The eluate was recovered and subjected to immunoassay to determine the Cav1/CD9 ratio as mentioned above.

Although the present invention has been described herein with reference to the specific embodiments, these embodiments do not serve to limit the invention and are set forth for illustrative purposes. It will be apparent to those skilled in the art that modifications and improvements can be made without departing from the spirit and scope of the invention.

Such simple modifications and improvements of the present invention belong to the scope of the present invention, and the specific scope of the present invention will be clearly defined by the appended claims.

### [Explanation of Reference Numerals]

1: Target of target subpopulation
10: Ligand
20: Binder
30: Recognition material
40: Immobilization member
50: Capture material
C: Conjugate
P: Target marker

## Claims

1. An affinity isolation system comprising ligands, binders to which the ligands reversibly bind and whose structural conformation is changed upon binding to the ligands, recognition materials specifically binding to the binders whose structural conformation has been changed, immobilization members whose surface is immobilized with either the binders or the recognition materials, and capture materials conjugated with the binders or recognition materials, not used in the immobilization, to form conjugates and specifically binding to targets of a target subpopulation in a sample solution.

2. The affinity isolation system according to claim 1, wherein the ligands are selected from the group consisting of sugar molecules, ions, substrates, antigens, and combinations thereof.

3. The affinity isolation system according to claim 1, wherein the binders are selected from the group consisting of sugar-binding proteins, ion-binding proteins, enzymes, antibodies, aptamers, cell receptors, nanostructures, and combinations thereof.

4. The affinity isolation system according to claim 1, wherein the recognition materials are selected from the group consisting of antibodies, protein receptors, cell receptors, aptamers, enzymes, nanoparticles, nanostructures, heavy metal chelators, and combinations thereof.

5. The affinity isolation system according to claim 1, wherein the immobilization members are selected from the group consisting of hydrogels, magnetic beads, latex beads, glass beads, nanometal structures, porous membranes, non-porous membranes, and combinations thereof.

6. The affinity isolation system according to claim 1, further comprising a reactor accommodating the immobilization members therein.

7. The affinity isolation system according to claim 1, wherein the binding complexes of the binders with the recognition materials are dissociated from each other when the ligands are detached from the binders.

8. The affinity isolation system according to claim 1, wherein the immobilization members bound to the targets are concentrated using magnetic force, gravitational force, and/or centrifugal force.

9. The affinity isolation system according to claim 1, wherein the sample solution comprises a body fluid and the targets are substances isolated from heterogeneous bulk populations in the body fluid.

10. The affinity isolation system according to claim 9, wherein the heterogeneous bulk populations comprise at least two substances selected from the group consisting of cells, exosomes, DNAs, RNAs, proteins, lipids, sugars, and metabolites used as biomarkers to diagnose target diseases.

11. An affinity isolation method comprising (a) serial reactions of a solution containing ligands, binders whose structural conformation is changed upon binding to the ligands, recognition materials specifically binding to the binders whose structural conformation has been changed, and capture materials conjugated with either the binders or the recognition materials to form conjugates such that the counterpart not used in the conjugation, *i.e.* the binders or recognition materials, bind to the conjugates, (b) addition of a sample solution containing targets of a target subpopulation capable of specifically binding to the capture materials such that the capture materials of the conjugates bind to the targets, and (c) addition of a recovery solution without the ligands such that the ligands are detached from the binders, triggering to dissociate the conjugates, formed the binding complexes with the targets, from the counterpart not used in the conjugation, *i.e.* the recognition materials or binders.

12. The affinity isolation method according to claim 11, wherein step (a) comprises immobilization of either of the binders or recognition materials, not used in the conjugation, on the surface of the immobilization members and addition of the ligand solution containing the conjugates dissolved therein to the immobilization members such that the conjugates bind to the counterpart immobilized onto the immobilization members.

13. The affinity isolation method according to claim 12, further comprising, between steps (b) and (c), capture of the immobilization members bound to the targets within predetermined spaces of a solution of the ligand mixed with the sample using magnetic force, gravitational force, and/or centrifugal force, and then removal of a portion of the mixed solution, where the immobilization members are absent, to enrich the targets.

14. The affinity isolation method according to claim 11, wherein the sample solution comprises a body fluid and the targets are substances isolated from heterogeneous bulk populations in the body fluid.

15. The affinity isolation method according to claim 14, wherein the heterogeneous bulk populations comprise at least two substances selected from the group consisting of cells, exosomes, DNAs, RNAs, proteins, lipids, sugars, and metabolites used as biomarkers for the diagnosis of target diseases.
